(19) Europäisches Patentamt
European Patent Office
Office européen des brevets

(11) **EP 4 424 306 A1**

(12) **EUROPEAN PATENT APPLICATION**
published in accordance with Art. 153(4) EPC

(43) Date of publication:
**04.09.2024 Bulletin 2024/36**

(21) Application number: **22886430.2**

(22) Date of filing: **22.08.2022**

(51) International Patent Classification (IPC):
*A61K 31/164* (2006.01)    *A23L 33/10* (2016.01)
*A61K 8/68* (2006.01)    *A61K 9/107* (2006.01)
*A61K 35/57* (2015.01)    *A61K 36/06* (2006.01)
*A61P 1/00* (2006.01)    *A61P 43/00* (2006.01)
*A61Q 19/08* (2006.01)

(52) Cooperative Patent Classification (CPC):
**A23L 33/10; A61K 8/68; A61K 9/107;
A61K 31/164; A61K 35/57; A61K 36/06;
A61P 1/00; A61P 43/00; A61Q 19/08**

(86) International application number:
**PCT/JP2022/031631**

(87) International publication number:
**WO 2023/074096 (04.05.2023 Gazette 2023/18)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.10.2021 JP 2021174062**

(71) Applicant: **Genuine R&D Co., Ltd.
Fukuoka-shi, Fukuoka 811-0215 (JP)**

(72) Inventors:
• **MIYANABE, Masakatsu
 Fukuoka-shi, Fukuoka 811-0215 (JP)**

• **HIRAKI, Shinobu
 Fukuoka-shi, Fukuoka 811-0215 (JP)**
• **ARAKAWA, Jun
 Fukuoka-shi, Fukuoka 811-0215 (JP)**
• **KATAKURA, Yoshinori
 Fukuoka-shi, Fukuoka 819-0395 (JP)**
• **TOKUMITSU, Yoshihiro
 Minamikyushu-shi, Kagoshima 897-0302 (JP)**
• **MATSUYAMA, Hiroyuki
 Minamikyushu-shi, Kagoshima 897-0302 (JP)**

(74) Representative: **Regimbeau
20, rue de Chazelles
75847 Paris Cedex 17 (FR)**

(54) **CERAMIDE COMPOSITION**

(57) A novel ceramide composition is provided. Specifically, an ethanol-extracted ceramide composition from chicken legs is provided.

[Fig. 8]

EP 4 424 306 A1

**Description**

Technical Field

The present disclosure relates to, for example, a ceramide composition and its use. The contents of all documents listed in the present specification are incorporated herein by reference.

Background Art

Ceramides, which are the main components of intercellular lipids in the human epidermis, have recently been expanding their market as, for example, functional cosmetic materials. It is known that stratum corneum ceramide levels gradually decrease with age, and supplementation of external ceramides is thus thought to be important for maintaining healthy skin. Since ceramides present as intercellular lipids in the stratum corneum are free ceramides, attention is also focused on free ceramides.

Studies have also been conducted so far to efficiently prepare useful ceramides (e.g., Patent Literature (PTL) 1 and PTL 2).

Citation List

Patent Literature

PTL 1: JP2012-126910A
PTL 2: WO2019/049964

Summary of Invention

Technical Problem

The present inventors further conducted research for the purpose of finding a novel function of ceramides.

Solution to Problem

The present inventors found the possibility that a specific organism-derived ceramide composition can have a specific function in a particularly efficient manner. Furthermore, among organism-derived ceramide compositions, the inventors found that a ceramide-containing extract obtained by ethanol extraction from chicken legs (ethanol-extracted ceramide composition from chicken legs) can have a particularly favorable effect of enhancing epithelial barrier functions. Based on such findings, the inventors made further improvements.

The present disclosure encompasses, for example, the subject matter described in the following items.

Item 1. An ethanol-extracted ceramide composition from chicken legs.
Item 2. The composition according to Item 1, comprising ω-O-acyl ceramide.
Item 3. The composition according to Item 2, wherein the w-O-acyl ceramide present in the composition is of type EOS, and the terminal acyl group is a linoleic acid group.
Item 4. The composition according to Item 2 or 3, wherein 0.1 to 2 mass% of the ceramide present in the composition is w-O-acyl ceramide.
Item 5. The composition according to any one of Items 1 to 4, which is for enhancing an epithelial barrier.
Item 6. The composition according to any one of Items 1 to 5, which is for enhancing a skin barrier or enhancing an intestinal barrier.
Item 7. The composition for enhancing a skin barrier according to Item 6, which is for taking care of skin damage caused by ultraviolet rays.
Item 8. The compositions for enhancing an intestinal barrier according to Item 6, which is for enhancing intestinal epithelial tight junctions.
Item 9. The composition according to any one of Items 1 to 8, wherein 90 mass% or more of the ceramide present in the composition is a free ceramide with a ceramide skeleton that is a combination of a sphingoid base containing 2 hydroxyl groups, 18 carbon atoms, and 1 carbon-carbon double bond; and a fatty acid containing 16 to 34 carbon atoms, 0 or 1 carbon-carbon double bond, and 0 hydroxyl groups.

Item 10. The composition according to any one of Items 1 to 8, wherein 90 mass% or more of the ceramide present in the composition is ceramide NS.

Advantageous Effects of Invention

A novel organism-derived ceramide composition is provided. In particular, a ceramide-containing extract obtained by ethanol extraction from chicken legs (an ethanol-extracted ceramide composition from chicken legs) is provided. The ceramide composition can be preferably used, in particular, for enhancing the epithelial barrier.

Brief Description of Drawings

Fig. 1 shows the results obtained of adding ceramides to HaCaT cells, culturing them for 48 hours, and then measuring the mitochondrial number, area, and membrane potential activity by using an IN Cell Analyzer 2200 (n=3; ***: $p < 0.001$, **: $p < 0.01$, *: $p < 0.05$; Student's t-test).
Fig. 2 shows the results obtained by adding ceramides to HaCaT cells irradiated with UVB at 10 mJ/cm$^2$, culturing them for 24 hours, and then measuring the ROS by using an IN Cell Analyzer 2200 (n=3; ***: $p < 0.001$, **: $p < 0.01$, *: $p < 0.05$; Student's t-test).
Fig. 3 shows the results obtained by adding ceramides to HaCaT cells irradiated with UVB at 10 mJ/cm$^2$, culturing them for 24 hours, and then measuring the mitochondrial number, area, and membrane potential activity by using an IN Cell Analyzer 2200 (n=3; ***: $p < 0.001$, **: $p < 0.01$, *: $p < 0.05$; Student's t-test).
Fig. 4 shows the results obtained by adding ceramides to HaCaT cells, culturing them for 48 hours, and then analyzing the fatty acid formulation in ceramides in the HaCaT cells by LC-MS.
Fig. 5 shows the results obtained by adding a supernatant of Caco-2 cells treated with ceramides for 24 hours to HaCaT cells irradiated with UVB at 10 mJ/cm$^2$, and after 48 hours, measuring the mitochondrial number, area, and membrane potential activity by using an IN Cell Analyzer 2200 (n=3; ***: $p < 0.001$, **: $p < 0.01$, *: $p < 0.05$, #=0.08; Student's t-test).
Fig. 6 shows the results obtained by adding a supernatant of Caco-2 cells treated with ceramides for 24 hours to HaCaT cells irradiated with UVB at 10 mJ/cm$^2$, and after 48 hours, measuring the ROS by using an IN Cell Analyzer 2200 (n=3; ***: $p < 0.001$, **: $p < 0.01$, *: $p < 0.05$, #: $p < 0.15$; Student's t-test).
Fig. 7 shows the results obtained by adding a supernatant of Caco-2 cells treated with ceramides for 24 hours to HaCaT cells irradiated with UVB at 10 mJ/cm$^2$, and after 48 hours, measuring the mitochondrial number, area, and membrane potential activity by using an IN Cell Analyzer 2200 (n=3; ***: $p < 0.001$, **: $p < 0.01$, *: $p < 0.05$; Student's t-test).
Fig. 8 shows the results obtained by adding ceramides to Caco-2 cells, culturing them for 24 hours, and then measuring the Claudin-3 mRNA expression levels (n=3; ***: $p < 0.001$, **: $p < 0.01$, *: $p < 0.05$, Student's t-test).

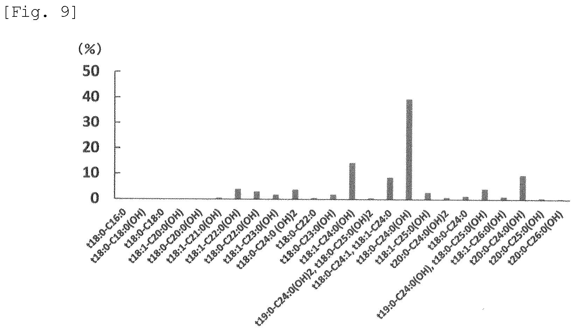

Fig. 9 shows the percentage (mass%) of various free ceramide species present in a ceramide composition extracted from soy sauce lees.

Description of Embodiments

Below, embodiments encompassed by the present disclosure are described in more detail. The present disclosure preferably encompasses a specific organism-derived ceramide composition, its use, and the like. However, the disclosure is not limited to these and encompasses all the matters disclosed herein and recognizable to those skilled in the art.
The ceramide composition encompassed by the present disclosure is an organism-derived ceramide composition, and is, more specifically, a ceramide composition extracted from soy sauce lees, a ceramide composition extracted from *Aspergillus oryzae,* or a ceramide composition extracted from chicken legs. The term "ceramide composition" means a composition comprising ceramide. The organism-derived ceramide may be referred to as "the ceramide composition of the present disclosure." The ceramide composition of the present disclosure is preferably a ceramide composition extracted from chicken legs. The ceramide composition extracted from chicken legs is particularly preferably a ceramide-containing extract obtained by ethanol extraction from chicken legs (an ethanol-extracted ceramide composition from chicken legs). The chicken legs are particularly preferably *momiji* (a portion of chicken feet). It is preferable to use the epidermal portion of chicken legs (in particular, *momiji*) for ethanol extraction. The chicken legs used for ethanol extraction are preferably dried in advance, and particularly preferably in dried-powder form.
The ceramide composition of the present disclosure, in particular, the ethanol-extracted ceramide composition from chicken legs, preferably has an effect of enhancing epithelial barrier functions. Therefore, the ceramide composition of the present disclosure can be preferably used for enhancing the epithelial barrier. In particular, preferable examples of the epithelial barrier include the skin barrier (epidermal barrier) and intestinal barrier (intestinal epithelial barrier).

When used for enhancing the skin barrier, the ceramide composition of the present disclosure, in particular, the ethanol-extracted ceramide composition from chicken legs, can be preferably used, in particular, for preventing effects (for taking care of damage) caused by ultraviolet rays on the skin. Without wishing to be bound by theory, this is because application of the ceramide composition of the present disclosure to the skin can enable restoration of mitochondria (in particular, the number, area, or membrane potential activity) in skin cells that will be damaged or have been damaged by ultraviolet rays. The ceramide composition of the present disclosure can be used for preventing skin damage caused by ultraviolet rays or for treating skin damage caused by ultraviolet rays.

When used for skin (when used for enhancing the skin barrier), the ceramide composition of the present disclosure can be used, for example, as an external composition or an oral composition (for example, an oral pharmaceutical composition or food composition), and preferably as an external composition.

Among the ceramide compositions of the present disclosure, in particular, the ethanol-extracted ceramide composition from chicken legs can be preferably used for the intestinal barrier, and particularly preferably for enhancing intestinal epithelial tight junctions. Without wishing to be bound by theory, this is because application of, in particular, the ethanol-extracted ceramide composition from chicken legs to the intestinal tract can enable an increase in the expression of claudins (in particular, claudin-3) in the cells in the intestinal tract. Enhancement of tight junctions can also enhance the defense against infection from pathogens in the intestinal tract. When used for enhancing the defense against infection, the composition can be used for prevention of infection or for treatment after infection. Claudins are major proteins involved in the formation of tight junctions and play a central role in the intercellular barrier at tight junctions.

For the density and integrity of tight junctions, transepithelial electric resistance (TER) can be measured as an index, and the ceramide composition of the present disclosure can improve the TER values in a preferable manner.

When used for the intestinal tract (used for the intestinal barrier), the ceramide composition of the present disclosure can be used, for example, as an oral composition (for example, an oral pharmaceutical composition or food composition) and a composition for intestinal administration. When used as a composition for intestinal administration, the composition can be administered directly into the intestine by a surgical procedure or through the anus.

Ceramides are compounds having a structure (-NH-CO-) in which the carboxyl group (-COOH) of a fatty acid is attached to the amino group ($-NH_2$) of a sphingoid base. Polar groups, such as sugar and phosphoric acid, are further attached to an alcoholic hydroxyl group (-OH) of the sphingoid base of ceramides to form a sphingoglycolipid and a sphingophospholipid, respectively. Ceramides to which sugar is attached are called, in particular, "glycosylceramides," and ceramides to which glucose is attached as the sugar are called, in particular, "glucosylceramides." Ceramides to which no sugar or phosphoric acid is attached are called, in particular, "free ceramides." When the fatty acid portion of a ceramide is an w-hydroxy fatty acid, and a further fatty acid is ester-bonded to the hydroxyl terminus, it is called, in particular, an "acyl ceramide (ω-O-acyl ceramide)." In the present specification, "acyl ceramide" means ω-O-acyl ceramide unless otherwise noted. The ceramide composition of the present disclosure preferably comprises an acyl ceramide.

Furthermore, examples of the fatty acid that is ester-bonded to the w-hydroxyl terminus include stearic acid, oleic acid, linoleic acid, and linolenic acid. Although not so particularly limited, the ceramide composition of the present disclosure (in particular, the ethanol-extracted ceramide composition from chicken legs) preferably comprises an acyl ceramide in which the fatty acid above is linoleic acid.

The sphingoid base constituting the free ceramide preferably has 2 or 3 hydroxyl groups, and more preferably 3 hydroxyl groups. Further, the sphingoid base preferably has 14 to 22 (14, 15, 16, 17, 18, 19, 20, 21, or 22) carbon atoms, more preferably 16 to 20 carbon atoms, and even more preferably 18 or 20 carbon atoms. The sphingoid base also preferably has 0 or 1 carbon-carbon double bond. More specific examples of preferable sphingoid bases include sphingosine, dihydrosphingosine, and phytosphingosine.

The fatty acid constituting the free ceramide preferably has 16 to 30 (16, 17, 18, 19, 20, 21, 22, 23, 24, 25, 26, 27, 28, 29, or 30) carbon atoms, more preferably 18 to 28 carbon atoms, even more preferably 20 to 26 carbon atoms, and still even more preferably 22 to 26 carbon atoms. The fatty acid also preferably has 0 or 1 carbon-carbon double bond, and more preferably 0 carbon-carbon double bonds (i.e., a saturated fatty acid). The fatty acid also preferably has 0, 1, or 2 hydroxyl groups. Although there is no particular limitation, when the fatty acid contains hydroxyl, the hydroxyl is preferably α-hydroxyl.

The combination of the sphingoid base and the fatty acid in the ceramide skeleton of the free ceramide may be any combination of sphingoid bases and fatty acids described above. Of these, a preferable example of the combination is a combination of a sphingoid base that contains 2 or 3 (in particular, 3) hydroxyl groups and a fatty acid that contains hydroxyl (in particular, α-hydroxyl). The number of hydroxyl groups in the fatty acid is preferably 0, 1, or 2.

Preferable examples include ceramide AS, which is a combination of sphingosine (S) and a fatty acid containing 1 hydroxyl group (A), and ceramide NS, which is a combination of sphingosine (S) and a fatty acid containing 0 hydroxyl group (N).

Preferable examples also include ceramide AP, which is a combination of phytosphingosine (P) and a fatty acid containing 1 hydroxyl group (A), and ceramide NP, which is a combination of phytosphingosine (P) and a fatty acid containing 0 hydroxyl groups (N). Further, ceramide DP, which is a combination of phytosphingosine (P) and a fatty acid containing 2 hydroxyl groups (D), is also a preferable example. "Ceramide AP" and "ceramide NP" are commonly used terms; however, "ceramide DP" is a term that is used in the present specification, and is not a commonly used term. Specific examples of ceramide DP include dihydroxylignoceroyl phytosphingosine and the like.

Among these, preferred as free ceramides are (i) free ceramides with a ceramide skeleton that is a combination of a sphingoid base containing 2 or 3 hydroxyl groups, 18 carbon atoms, and 0 or 1 carbon-carbon double bond; and a fatty acid containing 16 to 34 carbon atoms, 0 or 1 carbon-carbon double bond, and 0, 1, or 2 hydroxyl groups, and (ii) free ceramides with a ceramide skeleton that is a combination of a sphingoid base containing 2 or 3 hydroxyl groups, 20 carbon atoms, and no carbon-carbon double bonds; and a fatty acid containing 24 or 25 carbon atoms, 0 carbon-carbon double bonds, and 0, 1, or 2 hydroxyl groups. More preferred are the (i) free ceramides. Among these, furthermore preferred are (i-1) free ceramides with a ceramide skeleton that is a combination of a sphingoid base containing 2 hydroxyl groups, 18 carbon atoms, and 1 carbon-carbon double bond; and a fatty acid containing 16 to 34 carbons, 0 or 1 carbon-carbon double bond, and 0 hydroxyl groups, and (i-2) free ceramides with a ceramide skeleton that is a combination of a sphingoid base containing 3 hydroxyl groups, 18 carbon atoms, and 0 carbon-carbon double bonds; and a fatty acid containing 20 to 26 carbon atoms, 0 carbon-carbon double bonds, and 0 hydroxyl groups. For (i-1), particularly preferred are (i-1a) free ceramides with a ceramide skeleton that is a combination of a sphingoid base containing 2 hydroxyl groups, 18 carbon atoms, and 1 carbon-carbon double bond; and a fatty acid containing 16 to 34 carbon atoms, 0 carbon-carbon double bonds, and 0 hydroxyl groups.

More specifically, examples include d18:0-16:0, d18:0-18:0, d18:0-20:0, d18:0-22:0, d18:1-16:0, d18:1-18:0, d18:1-20:0, d18:1-22:0, d18:1-24:0, d18:1-26:0, d18:1-26:1, d18:1-28:0, d18:1-30:0, d18:1-32:0, d18:1-32:1, d18:1-34:0, d18:1-34:1, d18:0-16:0h, d18:0-18:0h, d18:0-20:0h, d18:0-22:0h, d18:1-16:0h, d18:1-18:0h, d18:1-20:0h, d18:1-22:0h, d18:1-24:0h, d18:1-26:0h, d18:1-28:0h, d18:1-30:0h, d18:1-32:0h, d18:1-34:0h, d18:1-34:1h, t18:0-23:0h, t18:1-23:0h, t18:0-24:0h, t18:1-24:0h, t20:0-24:0h, t18:1-26:0h, t18:0-25:0h, and t18:0-24:0h$_2$. This notation is explained using "t18:0-22:0h" as an example. The first half ("t18:0") is information about the sphingoid base and indicates a sphingoid base that contains 3 hydroxyl groups ("t"), 18 carbon atoms, and 0 carbon-carbon double bonds (i.e., containing no carbon-carbon double bonds). "d" indicates that 2 hydroxyl groups are present. The latter half ("22:0h") is information about the fatty acid and indicates a fatty acid that contains 22 carbon atoms, 0 carbon-carbon double bonds, and 1 hydroxyl group ("h"). "h$_2$" indicates that 2 hydroxyl groups are present. Additionally, "h" may be added immediately after the number of carbon atoms, as in "22h:0." Further, "h" may be not added, as in "22:0," when 0 hydroxyl groups are present (conversely, having no "h" means that 0 hydroxyl groups are present). It is also possible to add "c" before the number of carbon atoms, as in "c22:0."

The ceramide composition of the present disclosure is preferably an ethanol-extracted ceramide composition from chicken legs, which comprises an acyl ceramide. More specifically, the ethanol-extracted ceramide composition from chicken legs is preferably those comprising an acyl ceramide of d18:1-c28 to 36:0-18:2 (for example, d18:1-c28:0-18:2, d18:1-c30:0-18:2, d18:1-c32:0-18:2, d18:1-c34:0-18:2, or d18:1-c36:0-18:2), and particularly preferably those comprising 4 to 5 different acyl ceramides of these.

Ceramides, which constitute intercellular lipids of the human stratum corneum, have been classified by Downing et al. into seven classes of free ceramides according to the type of long-chain fatty acid that forms an amide linkage to a long-chain sphingoid base, and the formulations have also been reported (Robson K. J. et al., J. lipid Res. 35, 2060 (1994)). The acyl ceramide present in the ethanol-extracted ceramide composition from chicken legs is classified as ceramide EOS among those seven classes. Until now, an acyl ceramide that is prepared by chemical synthesis has been only ceramide EOP; however, ceramide EOP is rarely found in human skin, and an acyl ceramide that is known as human ceramide is actually ceramide EOS. Therefore, also from the perspective that ceramide EOS is present, the ethanol-extracted ceramide composition from chicken legs is preferable. The ceramide EOS is an acyl ceramide in which the sphingoid base portion is sphingosine, and the ceramide EOP is an acyl ceramide in which the sphingoid base portion is phytosphingosine.

The ethanol-extracted ceramide composition from chicken legs may be, for example, an acyl ceramide that is concentrated by HPLC or other known methods. In other words, the ethanol-extracted ceramide composition from chicken legs also encompasses acylamide concentrates of ethanol extracts from chicken legs.

Further, although not so particularly limited, the ethanol-extracted ceramide composition from chicken legs is preferably such that about 0.1 to 2 mass% of the ceramide present in the composition is an acyl ceramide. The upper limit or lower limit of this range (0.1 to 2 mass%) may be, for example, 0.2, 0.3, 0.4, 0.5, 0.6, 0.7, 0.8, 0.9, 1, 1.1, 1.2, 1.3, 1.4, 1.5, 1.6, 1.7, 1.8, or 1.9 mass%. For example, the range is more preferably 0.2 to 1.5 mass%, and even more preferably 0.3 to 1.2 mass%. These are preferable examples of the acyl ceramide content, and it can vary, for example, when the acyl ceramide is concentrated as stated above.

The ethanol-extracted ceramide composition from chicken legs comprises an acyl ceramide (preferably an acyl ceramide of d18:1-c28 to 36:0-18:2) as stated above, which is thought to play an extremely important functional role.

The acyl ceramide present in the ethanol-extracted ceramide composition from chicken legs is of type EOS, in which the sphingoid base is sphingosine and the fatty acid is an esterified ω fatty acid; additionally, the fatty acid esterified with the hydroxy group of the w fatty acid is mainly linoleic acid. The chain length of the w-hydroxy fatty acid portion is preferably such that 28 to 36 carbon atoms are present, and the center of the distribution is 30 to 34 carbon atoms.

It is well known that even a small amount of an acyl ceramide containing a very long-chain fatty acid group plays a central role in skin barrier functions. In particular, it is known that a decrease in acyl ceramide of type EOS, which is found in intercellular lipids of the human stratum corneum, not only causes a decrease in barrier functions and moisturizing functions, but also serves as an aggravating factor for skin barrier dysfunction and dry skin in, for example, atopic dermatitis.

In the ethanol-extracted ceramide composition from chicken legs, it is preferable that 90 mass% or more of the ceramide present in the composition is ceramide NS, and it is more preferable that 91, 92, 93, 94, 95, 96, 97, or 98 mass% or more thereof is ceramide NS. Further, in the ethanol-extracted ceramide composition from chicken legs, it is preferable that 90 mass% or more of the ceramide present in the composition is a ceramide in (i-1) above, and it is more preferable that 91, 92, 93, 94, 95, 96, 97, or 98 mass% or more thereof is a ceramide in (i-1) above. Furthermore, in the ethanol-extracted ceramide composition from chicken legs, it is preferable that 90 mass% or more of the ceramide present in the composition is a ceramide in (i-1a) above, and it is more preferable that 91, 92, 93, or 94 mass% or more thereof is a ceramide in (i-1a) above.

Although not so particularly limited, preferable examples of the ethanol-extracted ceramide composition from chicken legs include a composition comprising d18:1-20:0 in the highest amount, d18:1-22:0 in the second highest amount, and d18:1-26:0 in the third highest amount. For example, while maintaining the descending order of the amounts, the composition preferably comprises about 25 to 35 mass% (more preferably 27 to 33 mass%) of d18:1-20:0, about 15 to 30 mass% (more preferably 17 to 28 mass%) of d18:1-22:0, and about 5 to 20 mass% (more preferably 7 to 18 mass% or 8 to 15 mass%) of d18:1-26:0.

Further, the content of d18:1-16:0 is preferably 2 to 8 mass%, and more preferably 3 to 7 mass%. The content of d18:1-24:0 is preferably 4 to 11 mass%, and more preferably 5 to 10 mass% or 6 to 9 mass%. The content of d18:1-28:0 is preferably 2 to 8 mass%, and more preferably 3 to 7 mass%.

Furthermore, the content of d18:1-18:0 is preferably 2 to 6 mass%, and more preferably 3 to 5 mass%. The content of d18:1-26:1 is preferably 0.2 to 3 mass%, and more preferably 0.5 to 2 mass%. The content of d18:1-30:0 is preferably 1 to 4 mass%, and more preferably 2 to 3.5 mass%.

The ceramide composition of the present disclosure can also be used by nanoization (i.e., used as a nanoized ceramide composition). The nanoized ceramide composition can be obtained, for example, by subjecting the ceramide composition to high-pressure treatment. The high-pressure treatment can be performed with an ultra-high-pressure wet pulverization device (e.g., Star Burst, produced by Sugino Machine Limited). The high pressure is, for example, about 100 MPa or more, preferably about 100 to 300 MPa, and more preferably about 120 to 250 MPa.

The ceramide composition of the present disclosure can be preferably used as an emulsion composition. In particular, the form in which an oil phase is dispersed in an aqueous phase (e.g., an oil-in-water (O/W) emulsion) is preferred. In this case, in addition to ceramides extracted from organisms (in particular, ethanol-extracted ceramides from chicken legs), the composition can comprise oils and fats, nonionic surfactants, phospholipids, proteins, polysaccharides, pH adjusters, thickeners, fragrances, natural dyes, and preservatives.

When the composition is in the form of an O/W emulsion, the average particle size of the ceramide-containing oil droplets (i.e., dispersed particles) is preferably 500 nm or less. The average particle size of the dispersed particles is preferably 300 nm or less, more preferably 200 nm or less, and even more preferably 150 nm or less, in terms of stability during storage and bioavailability. The lower limit of the average particle size of the dispersed particles is not particularly limited and may be, for example, 1 nm or more, and preferably 10, 20, or 50 nm or more.

In the present disclosure, the average particle size of the dispersed particles means the volume average particle size measured using the dynamic-light-scattering method.

Examples of commercially available measurement devices that can measure the average particle size by the dynamic light scattering method include a Nanotrac WAVE II particle size distribution analyzer (Verder Scientific), a Zetasizer Ultra (Malvern Panalytical), an ELSZ-2000S particle size and molecular weight analyzer (Otsuka Electronics Co., Ltd.), and an SZ-100V2 nanoparticle analyzer (HORIBA, Ltd.).

In the present specification, the volume average particle size of the dispersed particles can be measured using, for example, a dynamic-light-scattering Nanotrac WAVE II (produced by Verder Scientific) and can be specifically measured as follows. A sample taken from the ceramide dispersion composition of the present invention is diluted with pure water so that the concentration of ceramide contained in the sample is 0.1 mass%, and measurement is performed using a quartz cell. The average particle size is determined as the volume average particle size (Mv) by setting the sample refractive index to 1.600 and the dispersion medium refractive index to 1.333 (pure water), and using the viscosity of pure water as the viscosity of the dispersion medium.

The average particle size of the ceramide-containing dispersed particles can also be adjusted by suitably adjusting the following conditions in the production method, such as high-pressure emulsification and dispersion conditions (number of passes, pressure, and temperature), stirring conditions (shearing force and temperature), the ratio of the oil phase to the aqueous phase, and other factors, in addition to the components of the composition.

To obtain an emulsion composition, the oil phase component and the aqueous phase component may be mixed and subjected to preliminary dispersion treatment to obtain a coarse dispersion in advance. Such means are not particularly limited, and a common stirrer can be used. Examples of stirrers include magnetic stirrers, household mixers, paddle mixers, impeller mixers, homomixers, disperser mixers, and ultramixers.

The time of the preliminary dispersion treatment is not particularly limited, and can be appropriately set according to, for example, the type of stirrer and the formulation of the liquid before the dispersion treatment.

The dispersion treatment step may be performed, for example, by subjecting the coarse dispersion obtained in the preliminary dispersion treatment (water or the like may be further mixed with the coarse dispersion, if necessary) to a dispersion treatment using an ultrasonic dispersion method (referred to below as "ultrasonic dispersion treatment") or a dispersion treatment using a high-pressure emulsification method (referred to below as "high-pressure emulsification treatment").

In the dispersion treatment step, the coarse dispersion containing the organism-extracted ceramides of the present invention (water or the like may be further mixed with the coarse dispersion, if necessary) is subjected to a dispersion treatment to obtain a fine-particle ceramide dispersion composition comprising dispersed particles containing the organism-extracted ceramides.

The dispersion treatment in the main dispersion step is preferably a high-pressure emulsification treatment, in terms of reducing the size of the dispersed particles.

The high-pressure emulsification treatment refers to a dispersion treatment in which a shearing force of 10 MPa or more is applied to a material to be dispersed. To reduce the size of the dispersed particles, the shearing force applied to the material to be dispersed is preferably 50 or 100 MPa or more, and more preferably 150 MPa or more. The upper limit is preferably, for example, 300 MPa or less in terms of temperature increase and pressure resistance.

The means for the high-pressure emulsification treatment is not particularly limited, and a general-purpose high-pressure emulsification device can be used. Examples of high-pressure emulsification devices include high-pressure homogenizers, such as a Star Burst HJP-25005 (produced by Sugino Machine Limited), a Microfluidizer (produced by Microfluidics), Nano-Mizer (produced by Yoshida Kikai Co., Ltd.), a Gaulin homogenizer (produced by APV), a Rannie homogenizer (produced by Rannie), a high-pressure homogenizer (produced by Niro Soavi), a homogenizer (produced by Sanwa Machinery Trading Co., Ltd.), a high-pressure homogenizer (produced by Izumi Food Machinery), and an ultra-high-pressure homogenizer (produced by IKA).

The temperature during the high-pressure emulsification treatment is preferably set to 30 to 80°C, and more preferably 40 to 70°C.

Although the high-pressure emulsification treatment may be performed once, the high-pressure emulsification treatment is preferably performed two or more times, and more preferably two to five times, in order to increase the homogeneity of the entire liquid. To maintain the particle size of the dispersed particles, it is preferable that the emulsified liquid, which is a composition that has undergone emulsification and dispersion, is cooled through some sort of cooler within 30 seconds, and preferably within 3 seconds, immediately after passing through the chamber.

The production method may comprise other steps as necessary, in addition to the preliminary dispersion treatment step and the main dispersion treatment step. Examples of the other steps include a heat sterilization step.

Furthermore, the emulsion may be subjected to the step of drying to thus obtain a powder composition. Powdering has many benefits, such as improving portability and reducing transportation costs, in addition to improving long-term storage by removing the water content. The ceramide composition of the present disclosure also preferably encompasses such a powder composition.

The drying means for use may be known drying means. Examples include natural drying, heat drying, hot-air drying, high-frequency drying, ultrasonic drying, reduced-pressure drying, vacuum drying, freeze drying, and spray drying. These means may be used alone or in a combination of two or more.

In order to prevent agglomeration and coalescence of hydrophobic particles during the drying process, an inclusion agent may be present together. Polysaccharides and oligosaccharides that are easily soluble in water are preferable as the inclusion agent. The saccharides used as a saccharide inclusion agent in the present invention are preferably those composed of sugar units as the basic unit, and the average polymerization degree of the sugar units (the number of sugar units) is usually preferably 60 or less, and more preferably 2 to 50, in terms of particle size refinement after condensation. For use as such a saccharide inclusion agent, preferred are inulin, raffinose, stachyose, verbascose, and trehalose, in terms of the effect of stabilizing the coalescence of hydrophobic particles.

In the present invention, spray drying is particularly preferred as a drying means so as to improve both the production efficiency and quality. Spray drying is a type of convection hot-air drying. An emulsion in liquid form is sprayed as minute particles of several hundred micrometers or less into hot air and is dried as it falls through the tower to be collected as a solid powder. Although the material is temporarily exposed to hot air, the exposure time is very short, and the temperature does not rise much because of the latent heat of vaporization of the water; thus, like freeze drying, thermal denaturation of the material is unlikely to occur, and changes due to condensation are also small. If the material is very heat-sensitive, supplying cold air instead of hot air is also possible, which is preferable because milder drying can be achieved although the drying power is reduced.

Examples of commercially available spray dryers include, but are not limited to, an SD-1000 spray dryer (Tokyo Rikakikai Co., Ltd.), an L-8i spray dryer (Ohkawara Kakohki Co., Ltd.), a CL-12 closed spray dryer (Ohkawara Kakohki Co., Ltd.), an ADL310 spray dryer (Yamato Scientific Co., Ltd.), a B-290 mini spray dryer (Buchi), PJ-MiniMax (Powdering Japan K.K.), and PharmaSD (GEA Process Engineering Inc.).

It is also preferable, for example, to use devices that can perform drying and granulation at the same time, such as an MP-01 fluidized bed granulation dryer (Powrex Corporation) and an FSD built-in fluidized spray dryer (GEA Process Engineering Inc.), to obtain granules with excellent handleability simultaneously with drying.

The ceramide composition of the present disclosure can be used as an external composition, an oral composition, a composition for intestinal administration, and the like, as stated above. The composition can also be preferably used in the fields of pharmaceuticals and foods. The composition may consist of ceramide or may comprise ceramide and other components (various bases, carriers, additives, etc.), as described below in detail. The composition also encompasses a ceramide-containing biological tissue extract itself. In other words, a ceramide-containing biological tissue extract (optionally further containing other components) can also be used as the ceramide composition of the present disclosure.

When the ceramide composition of the present disclosure is used in the field of pharmaceuticals (including medicaments and quasi-drugs), the composition (which may be referred to below as "the pharmaceutical composition of the present disclosure") may be a pharmaceutical composition consisting of each organism-extracted ceramide or also comprising other components. For example, the pharmaceutical composition of the present invention comprises ceramide as an active ingredient and can optionally comprise pharmaceutically acceptable bases, carriers, additives (e.g., excipients, binders, disintegrants, lubricants, solvents, sweeteners, colorants, flavoring agents, smell-masking agents, surfactants (especially emulsifiers), humectants, preservatives, pH adjusters, and thickening agents), and the like. Such bases, carriers, additives, and the like are specifically described, for example, in the Dictionary of Pharmaceutical Additives 2016 (Yakuji Nippo, Limited), and those described therein, for example, can be used. The formulation form is also not particularly limited. The active ingredient and other components may be mixed according to commonly used methods to formulate the mixture into a formulation, such as an oral composition. Examples of oral compositions include tablets, coated tablets, powders, granules, fine granules, capsules, pills, liquid agents, suspensions, emulsions, jellies, chewable agents, and soft tablets. For example, tablets can be produced by a tableting method. Both a direct tableting method, in which a raw material mixture is directly compressed into tablets, and a granulation tableting method, in which a raw material mixture is granulated and then compressed into tablets, can be used. Further, for example, the capsules may be soft capsules or hard capsules. Further, for example, the external composition can also be used as a cream, a milky lotion, a gel agent, and the like. Further, for example, the composition for intestinal administration can be used, for example, as a foam agent.

The amount of the ceramide contained in the pharmaceutical composition of the present invention is not particularly limited and can be set as appropriate depending on the subject as long as an anti-fatigue effect is achieved. The amount is preferably 0.0005 to 100 mass%, more preferably 0.005 to 90 mass%, and even more preferably 0.05 to 80 mass%. The lower limit may be about 10 mass%, 20 mass%, 30 mass%, 40 mass%, 50 mass%, 60 mass%, 70 mass%, or 80 mass%.

The subjects to which the pharmaceutical composition of the present disclosure is administered include not only humans but also non-human mammals. Examples include mammals that suffer from fatigue. In particular, mammals kept as pets and livestock are preferred. Specific examples include dogs, cats, monkeys, cows, horses, sheep, goats, pigs, rabbits, mice, rats, camels, and llamas. For mammals, the pharmaceutical composition of the present invention can also be used prophylactically, as it is for humans.

The timing for administration of the pharmaceutical composition of the present disclosure is not particularly limited and can be suitably selected, for example, in consideration of the formulation form, the age of the subject for administration, the severity of symptoms of the subject for administration, and the like.

The amount for administration of the pharmaceutical composition of the present disclosure can be suitably selected according to, for example, the age of the subject for administration, the severity of symptoms of the subject for administration, and other conditions. The amount can be suitably set, in particular, based on the amount of the ceramide contained as long as the effects of the present invention are not impaired. The administration can be performed once or divided multiple times (preferably two to three times) per day. For mammals as well, the amount can be suitably set with reference to the cases for humans.

When the ceramide composition of the present disclosure is used as a food composition (for example, a food and drink product or a food additive), the composition (which may be referred to below as "the food composition of the present disclosure") comprises ceramide, and appropriately comprises, for example, food-hygienically acceptable bases, carriers, additives, and other components and materials that can be used as food and drink products. Examples of such a food composition include processed foods, drinks, health foods (e.g., foods with nutrient function claims and foods for specified health uses), supplements, and medical foods (e.g., hospital foods, foods for the sick, and nursing care foods), all of which comprise ceramide and are for improving or preventing fatigue. Although not so limited, if the ceramide contained in the food composition is a biological tissue-extracted ceramide that is extracted from tissue of organisms (in particular, animals or plants), the food composition is preferably, for example, a processed food, a health food (e.g., food with nutrient function claims and food for specified health uses), a supplement, a medical food, or the like, all of which comprise the ceramide. Further, ceramides may be made into, for example, a powder form and blended in various food and drink products, such as drinks (e.g., juices), confectioneries, breads, soups (including powdered soups etc.), and processed foods.

When the food composition of the present invention is prepared as a health food (e.g., food with nutrient function claims and food for specified health uses) or a supplement, the food composition is preferably prepared in the form of, for example, granules, capsules, tablets (including chewable agents etc.), and drinks (drink preparations) so as to facilitate continuous consumption, and more preferably prepared in the form of capsules and tablets from the perspective of ease of consumption. However, the form is not particularly limited to these. The food composition of the present invention in the form of granules, capsules, tablets, etc. can be suitably prepared according to common methods by using pharmaceutically and/or food-hygienically acceptable carriers and the like. The preparation into other forms can also be performed according to common methods.

The amount of the ceramide in the food composition of the present disclosure is not particularly limited as long as the effects of the composition are obtained. The amount is preferably 0.0005 to 100 mass%, more preferably 0.005 to 90 mass%, and even more preferably 0.05 to 80 mass%. The lower limit may be about 10 mass%, 20 mass%, 30 mass%, 40 mass%, 50 mass%, 60 mass%, 70 mass%, or 80 mass%.

The amount for consumption, the subject for consumption, etc. of the food composition of the present disclosure are not particularly limited and are preferably, for example, the same as those for the pharmaceutical composition of the present invention described above.

The hospital foods are meals served when a patient is admitted to a hospital, the foods for the sick are meals for the sick, and the nursing care foods are meals for care recipients.

In the present specification, the terms "comprising" and "containing" include "consisting essentially of" and "consisting of." The present disclosure covers all combinations of the elements described in the present specification.

In addition, the various characteristics (properties, structures, functions, etc.) described in each embodiment of the present disclosure described above may be combined in any way in specifying the subject matter included in the present disclosure. In other words, the present disclosure includes all the subject matter comprising all combinations of the combinable characteristics described in the present specification.

Examples

The present invention is described in more detail below. However, the present invention is not limited to the following Examples.

Preparation of Ceramide Compositions

Ceramide Composition Extracted from Soy Sauce Lees

A ceramide composition was obtained by subjecting soy sauce lees to ethanol extraction and purifying high-purity free ceramides by solvent fractionation with reference to the method described in JP2012-126910A.

More specifically, the ceramide composition was prepared in the following manner. That is, a dried product of pressed lees, which is a by-product of soy sauce produced according to a common method, was subjected to ethanol extraction, and the obtained extract was subjected to solid-liquid separation using ethanol and water to obtain solids. Further, the solids were washed with acetone, ethanol, and water, dried, and pulverized, followed by washing with water, acetone, and hexane. Ethanol extraction was then performed again to obtain solids as a ceramide composition.

Ceramide Composition Extracted from *Aspergillus oryzae*

Fungal cells were collected from 300 liters of an *Aspergillus oryzae* culture solution and dried to obtain a solid material. A 2.5-fold amount of an ethanol-water mixture was added to the solid material and mixed, followed by extraction with stirring at 40°C overnight. After collecting the extract, a 1.2-fold amount of an ethanol solution was added to the solids and mixed, followed by extraction with stirring at 40°C overnight. The obtained extracts were combined and distilled under reduced pressure at 45°C to obtain a concentrated extract. After the concentrated extract was washed with acetone (and further washed with ethanol, acetone, etc., if necessary), the resulting insoluble matter was used as a ceramide composition extracted from *Aspergillus oryzae*.

Ceramide Composition Extracted from Chicken Legs

A 2.5-fold amount of 95% ethanol was added to a dried chicken material and mixed, followed by extraction with stirring at 40°C overnight. After collecting the extract, a 1.2-fold amount of ethanol was added to the solids and mixed, followed by extraction with stirring at 40°C overnight. The obtained extracts were combined and distilled under reduced pressure at 45°C to obtain a concentrated extract. After the concentrated extract was washed with acetone (and further washed with ethanol, acetone, etc., if necessary), the resulting insoluble matter was used as a ceramide composition extracted from chicken legs. The ceramide composition extracted from chicken legs can be referred to, more specifically, as an "ethanol-extracted ceramide composition from chicken legs."

Formulation Analysis of Ceramide Composition by LC-MS

Each ceramide composition was analyzed by LC-MS (high-performance liquid chromatography mass spectrometry), and various free ceramide species contained in each ceramide composition were analyzed.

Fig. 15 and Table 1 show the percentages (mass%) of various free ceramide species in the ceramide composition extracted from soy sauce lees. Three free ceramide species, i.e., t18:0-24:0h, t18:1-24:0h, and t20:0-24:0h, made up not less than 50 mass% of the ceramide composition. It was also found that the ceramide composition contained other free ceramides.

[Table 1]

| Molecular species | Ceramide extracted from soy sauce lees |
|---|---|
| | Relative value |
| t18:0-C16:0 | 0.13 |
| t18:0-C18:0 | 0.13 |
| t18:0-C22:0 | 1.44 |
| t18:0-C24:0 | 3.38 |
| t18:0-C18:0(OH) | 0.12 |
| t18:0-C20.0(OH) | 0.27 |
| t18:0-C22:0(OM) | 7.71 |
| t18:0-C23:0(OH) | 4.86 |
| t18:0-C24:0(OH) | 100 |
| t18:0-C25:0(OM) t19:0-C24:0(OH) | 10.63 |
| t18:0-C24:0(OH)$_2$ | 977 |
| t18:0-C25:0(OH)$_2$ t19:0-C24:0(OH)$_2$ | 1.35 |
| t18:1C20:0(OH) | 0 |
| t18:1-C21:0(OH) | 1.42 |
| t18:1-C22:0(OH) | 10.26 |
| t18:1-C23:0(OH) | 4.57 |
| t18:1-C24:0(OH) | 36.34 |
| t18:1-C25:0(OH) | 7.00 |
| t18:1-C26:0(OH) | 2.79 |
| t20:0-C24:0(OH) | 24.35 |
| t20:0-C25:0(OH) | 1.47 |
| t20:0-C26:0(OH) | 0.62 |
| t20:0-C24:0(OH)$_2$ | 2.16 |

Table 2 shows free ceramide molecular species in the ceramide composition extracted from *Aspergillus oryzae*.

[Table 2]

| Free ceramide molecular species | Percentage (%) | |
|---|---|---|
| t18:0 - 24:0 | 10.78 | |
| 26:0 | 0.13 | |
| 24h:0 | 34.57 | |
| 26h:0 | 3.75 | |
| t19:0 - 24:0 | 0.50 | |
| 24h:0 | 1.99 | |
| 26h:0 | 0.22 | |
| t20:0 - 24:0 | 1.66 | |
| 22h:0 | 0.06 | |
| 24h:0 | 44.82 | |
| 26h:0 | 1.52 | |

For the ceramide composition extracted from chicken legs, four lots were prepared to analyze various free ceramide species contained. Table 3a and Table 3b show the results. Table 3a shows the contents (mg/g) of various free ceramides, and Table 3b shows the concentrations (mass%) of various free ceramides.

[Table 3a]

| | Lot No. 1 | Lot No. 2 | Lot No. 3 | Lot No. 4 | |
|---|---|---|---|---|---|
| Free ceramide species | (mg/g) | (mg/g) | (mg/g) | (mg/g) | |
| d18:1-c16:0 | 4.7 | 26.9 | 4.6 | 4.3 | |
| d18:1-c28:0 | 3.8 | 18 | 3.2 | 5.1 | |
| d18:1-c20:0 | 30.2 | 117.8 | 26.6 | 41.9 | |
| d18:1-c22:0 | 21.8 | 81.1 | 22.2 | 32.8 | |
| d18:1-c24:0 | 7.2 | 26.8 | 7 | 9 | |
| d18:1-c26:1 | 1.3 | 3.9 | 0.9 | 2.1 | |
| d18:1-c26:4 | 13.6 | 36.4 | 9.7 | 17.7 | |
| d18:1-c28:0 | 6.5 | 17.1 | 2.9 | 7.6 | |
| d18:1-c30:0 | 2.4 | 9.3 | 2.3 | 4.7 | |
| d18:1-c32:1 | 0.9 | 8 | 1.5 | 2.3 | |
| d18:1-c32:0 | 2.1 | 6.5 | 1 | 2.6 | |
| d18:1-c34:1 | 1.7 | 11.8 | 1.8 | 2.6 | |
| d18:1-c34:0 | 0.9 | 26.3 | 0.7 | 1.2 | |
| t18:0-c20:0 | 0.9 | | | | |
| t18:0-c22:0 | 2.8 | | | | |
| t18:0-c24:0 | 1 | | | | |
| t18:0-c26:0 | 0.4 | | | | |
| d18:1-c28:0-18:2 | | 0.3 | 0.1 | | |
| d18:1-c30:0-18:2 | 0.1 | 0.6 | 0.1 | 0.2 | |

(continued)

|  | Lot No. 1 | Lot No. 2 | Lot No. 3 | Lot No. 4 |  |
|---|---|---|---|---|---|
| Free ceramide species | (mg/g) | (mg/g) | (mg/g) | (mg/g) |  |
| d18:1-c32:0-18:2 | 0.1 | 0.7 | 0.1 | 0.2 |  |
| d18:1-c34:0-18:2 | 0.1 | 0.5 | 0.1 | 0.2 |  |
| d18:1-c36:0-18:2 | 0.1 | 0.6 | 0.1 | 0.1 |  |

[Table 3b]

|  | Lot No. 1 | Lot No. 2 | Lot No. 3 | Lot No. 4 |  |
|---|---|---|---|---|---|
| Free ceramide species | (mass%) | (mass%) | (mass%) | (mass%) |  |
| d18:1-c16:0 | 4.58 | 6.85 | 5.42 | 3.19 |  |
| d18:1-c18:0 | 3.70 | 4.58 | 3.77 | 3.79 |  |
| d18:1-c20:0 | 29.43 | 30.01 | 31.33 | 31.13 |  |
| d28:1-c22:0 | 21.25 | 20.66 | 26.15 | 24.37 |  |
| d18:1-c24:0 | 7.02 | 6.83 | 8.24 | 6.69 |  |
| d18:1-c26:1 | 1.27 | 0.99 | 1.06 | 1.56 |  |
| d18:1-c26:0 | 13.26 | 9.27 | 11.43 | 13.15 |  |
| d18:1-c28:0 | 6.34 | 4.36 | 3.42 | 5.65 |  |
| d18:1-c30:0 | 2.34 | 2.37 | 2.71 | 3.49 |  |
| d18:1-c32:1 | 0.88 | 2.04 | 1.77 | 1.71 |  |
| d18:1-c32:0 | 2.05 | 1.66 | 1.18 | 1.93 |  |
| d18:1-c34:1 | 1.66 | 3.01 | 2.12 | 1.93 |  |
| d18:1-c34:0 | 0.88 | 6.70 | 0.82 | 0.89 |  |
| t18:0-c20:0 | 0.88 | 0.00 | 0.00 | 0.00 |  |
| t18:0-c22:0 | 2.73 | 0.00 | 0.00 | 0.00 |  |
| t18:0-c24:0 | 0.97 | 0.00 | 0.00 | 0.00 |  |
| t18:0-c2G:0 | 0.39 | 0.00 | 0.00 | 0.00 |  |
| d18:1-c28:0-18:2 | 0.00 | 0.08 | 0.12 | 0.00 |  |
| d18:1-c30:0-28:2 | 0.10 | 0.15 | 0.12 | 0.15 |  |
| d18:1-c32:0-18:2 | 0.10 | 0.18 | 0.12 | 0.15 |  |
| d18:1-c34:0-18:2 | 0.10 | 0.13 | 0.12 | 0.15 |  |
| d18:1-c36:0-18:2 | 0.10 | 0.15 | 0.12 | 0.07 |  |

In the tables, "d18:1-c28:0-18:2," "d18:1-c30:0-18:2," "d18:1-c32:0-18:2," "d18:1-c34:0-18:2," and "d18:1-c36:0-18:2" represent acyl ceramides.

Nanoization of Ceramide

Glycols (optionally other polyols, fats and oils, etc. may also be added), an emulsifier, and a ceramide composition were mixed and heated to 100 to 150°C (a temperature near the melting point of ceramide), and the mixture was homogenized. An aqueous lecithin dispersion, a polysaccharide aqueous solution, a water-soluble polymer aqueous solution, etc. were further added to the homogenized mixture, followed by mixing with stirring. The resulting mixture was treated at a high pressure of 100 MPa or more with an ultra-high-pressure wet pulverization device (Star Burst, produced by Sugino Machine Limited) to nanoize the ceramide. The lecithin was used in an amount of about 2 to 5 times that of the ceramide on a mass ratio basis.

The particle size of the ceramide-containing particles of the obtained nanoized ceramide composition was measured using a dynamic-light-scattering Nanotrac WAVE II (Verder Scientific). Specifically, the nanoized ceramide composition (emulsion) was diluted with pure water so that the ceramide concentration was 0.1 mass%, and the thus-obtained product was used as a measurement sample. The volume average particle size (Mv) was determined by setting the sample refractive index to 1.600 and the dispersion medium refractive index to 1.333 (pure water), and using the viscosity of pure water as the viscosity of the dispersion medium. The ceramide particle size of the ceramide composition before the nanoization treatment was also measured in a similar manner. The volume average particle size of the ceramide in the ceramide composition before the nanoization treatment was 4857 nm, and the volume average particle size of the ceramide in the nanoized ceramide composition was 124 nm.

Below, a product obtained by subjecting the ceramide composition extracted from soy sauce lees to nanoization was used as the nanoized ceramide composition.

The ceramide compositions (the ceramide composition extracted from soy sauce lees, the ceramide composition extracted from *Aspergillus oryzae,* the ceramide composition extracted from chicken legs, and the nanoized ceramide composition), as well as glucosylceramide and synthetic ceramide, were used as ceramide samples. In the following analysis, the ceramide concentration refers to the concentration of each ceramide sample. Below, the phrase "xxx-extracted ceramide composition" or "ceramide composition extracted from xxx" may be abbreviated as "xxx ceramide." For example, a ceramide composition extracted from soy sauce lees may be expressed as "soy sauce lees ceramide." Further, a ceramide composition extracted from chicken legs may be referred to as "avian." For the glucoside ceramide, a pinecera powder, produced by Maruzen Pharmaceuticals Co., Ltd., was used. For the synthetic ceramide, ceramide NG (Ceramide TIC-001), produced by Takasago International Corporation, was used. For NAC, N-acetyl-L-cysteine, produced by FUJIFILM Wako Pure Chemical Corporation, was used.

Analytical Method

Evaluation of Barrier Function Enhanced by Ceramide in HaCaT Cells

HaCaT Cell Culture

A human epidermal keratinocyte cell line, i.e., HaCaT cells, was used as a human skin cell model. The cells were subcultured in a cell culture dish (Greiner Bio-One Co., Ltd., Tokyo, Japan) at 37°C in the presence of 5% $CO_2$ by using DMEM medium (Dulbecco's Modified Eagle Medium, Nissui, Tokyo, Japan) containing 10% inactivated fetal bovine serum (FBS, Life Technologies Corporation, CA, USA). The DMEM medium was prepared by dissolving 4.75 g of Dulbecco's Modified Eagle Medium (DMEM) medium Nissui (2) (produced by Nissui Pharmaceutical Co., Ltd., Tokyo, Japan) in 470 mL of Milli-Q water, and adding 10 mL of 0.2 M L-glutamine (FUJIFILM Wako Pure Chemical Corporation, Osaka, Japan), 1 mL of 50000 U/mL penicillin (Meiji Seika Pharma Co., Ltd., Tokyo, Japan), 0.5 mL of 0.05 mg/mL streptomycin (Meiji Seika Pharma Co., Ltd.), and 6 mL of 10% $NaHCO_3$ (FUJIFILM Wako Pure Chemical Corporation).

Measurement of Mitochondrial Activity by Using IN Cell Analyzer 2200

HaCaT cells were seeded in a 96-well plate at a density of $6.0 \times 10^4$ cells/mL and precultured for 24 hours. The samples were then added thereto so that the final ceramide concentration was 5 $\mu$M. After 24 hours, 100 $\mu$L/well of MitoTracker Red (Invitrogen, USA) diluted to 2.5 $\mu$M with 10% FBS/DMEM was added to the plate after removing the medium, followed by incubation at 37°C and 5% $CO_2$ for 30 minutes. After 30 minutes, the medium containing MitoTracker Red was removed, and 100 $\mu$L/well of MitoTracker Green (Invitrogen) diluted to 200 nM with 10% FBS/DMEM was added, followed by incubation at 37°C and 5% $CO_2$ for 30 minutes. Subsequently, 100 $\mu$L/well of a solution obtained by 500-fold dilution of 1 mg/mL of Cellstain Hoechst 33342 solution (Dojindo Laboratories, Kumamoto, Japan) with DMEM was added, followed by incubation at 37°C and 5% $CO_2$ for 30 minutes. Thereafter, the diluted Hoechst 33345 solution was removed from the 96-well plate, 150 mL of PBS was added, and the fluorescence was detected using an IN Cell Analyzer 2200 (GE Healthcare). By measuring the amounts of fluorescence of MitoTracker Red, MitoTracker Green, and Hoechst 33342, the mitochondrial activity, number, and area in the cells were examined. The images were analyzed using IN Cell Investigator High-content image analysis software (GE Healthcare).

ROS Measurement by Using IN Cell Analyzer 2200

Subconfluent HaCaT cells seeded in a 10-mL dish were irradiated with UVB at 10 mJ/cm$^2$ in a UV crosslinker (CL-1000 Ultraviolet Crosslinker, UVP, Upland, CA, USA) with the lid of the dish removed. Immediately thereafter, 10 mL of DMEM medium containing 10% FBS was added, followed by incubation at 37°C in the presence of 5% $CO_2$. Untreated cells were not irradiated with UVB, and only replacement of the medium was performed. After 24 hours, the culture solutions of non-UVB-irradiated cells and UVB-irradiated cells were removed, and then the cells were washed with 1×PBS, detached with trypsin, and seeded in a 96-well plate at $3 \times 10^3$ cells per well. After 4 hours, the cells were subjected to ceramide treatment and incubated for 24 hours. Thereafter, intracellular ROS production in the HaCaT cells was measured according to the manufacturer's protocol by using BES-$H_2O_2$-AC (Wako Pure Chemical Industries, Ltd.). The culture solution in the 96-well plate was removed, and the cells were washed twice with 4-(2-hydroxyethyl)-1-piperazineethanesulfonic acid (HEPES) buffer (pH of 7.4). A CellstainR Hoechst 33342 solution (Dojindo Laboratories) was mixed with 1×PBS at a ratio of 1:500 and applied to the cells, and the resulting product was allowed to stand at room temperature for 30 minutes while being protected from light. Thereafter, 5 $\mu$M of BES-$H_2O_2$-AC in HEPES (pH of 7.4) was mixed at 1:1000, and Hoechst 33342 (Dojindo Laboratories) was mixed at 1:400, followed by incubation with the cells for 60 minutes at 37°C. The protocol used was HaCaT_eGFP20190627, and images were acquired with an IN Cell Analyzer 2200 (GE Healthcare). The images were analyzed with an IN Cell Analyzer 2200 Workstation (GE Healthcare), and the ROS expression level was examined.

Effect of Ceramide on Repair of UV Damage in UVB-induced Senescent Cells

An experiment similar to that in the "Measurement of Mitochondrial Activity by Using IN Cell Analyzer 2200" section was performed using subconfluent cells irradiated with UVB at 10 mJ/$cm^2$.

Evaluation of Human Ceramide Synthesis in HaCaT Cells

The samples were added to subconfluent HaCaT cells cultured in a 10-mL dish to a final ceramide concentration of 5 $\mu$M. After 48 hours, the medium was removed, washing was performed with 5 ml of 1×PBS, and 1.5 ml of trypsin was spread over the entire dish and removed. The cells were then incubated at 37°C and 5% $CO_2$ for 8 minutes, detached with DMEM, collected in a 15-mL tube, and centrifuged at 12,000 rpm for 3 minutes to collect cell pellets. The fatty acid formulation in the ceramides contained in the HaCaT cells was analyzed by LC-MS.

Evaluation of Influence of Ceramide on HaCaT Cells via Caco-2 Cells and Analysis of Barrier Function in the Intestinal Tract Caco-2 Cell Culture

A human colon cancer-derived cell line, i.e., CaCo-2 cells, was used as a human intestinal epithelial cell model. The CaCo-2 cells were subcultured in a cell culture dish (Greiner Bio-One Co., Ltd., Tokyo, Japan) at 37°C in the presence of 5% $CO_2$ by using DMEM medium containing 10% inactivated fetal bovine serum (FBS) (in which the complement had been inactivated by heating in a constant temperature water bath at 56°C for 35 minutes). The DMEM medium was prepared by dissolving 4.75 g of Dulbecco's Modified Eagle Medium (DMEM) medium Nissui (2) (produced by Nissui Pharmaceutical Co., Ltd., Tokyo, Japan) in 470 mL of Milli-Q water, and adding 10 mL of 0.2 M L-glutamine (FUJIFILM Wako Pure Chemical Corporation, Osaka, Japan), 1 mL of 50000 U/mL penicillin (Meiji Seika Pharma Co., Ltd., Tokyo, Japan), 0.5 mL of 0.05 mg/mL streptomycin (Meiji Seika Pharma Co., Ltd.), and 6 mL of 10% $NaHCO_3$ (FUJIFILM Wako Pure Chemical Corporation).

Measurement of Mitochondrial Activity by Using IN Cell Analyzer 2200 (Caco-2 Cells → HaCaT Cells)

Caco-2 cells were seeded at $1.0 \times 10^2$ cells/mL in a 24-well plate (Corning Incorporated, NY, USA). After 24 hours, ceramides were added to a final concentration of 5 $\mu$M, and culture was performed for 24 hours. HaCaT cells were seeded at $6.0 \times 10^4$ cells/mL in a 96-well plate and incubated for 24 hours, and 50 $\mu$L of the HaCaT cell culture solution was removed. To this culture solution, 50 $\mu$L of the Caco-2 cell culture solution was added. After 48 hours, the mitochondrial number and area, as well as the mitochondrial membrane potential activity, in the HaCaT cells were measured in the same manner as in the "Measurement of Mitochondrial Activity by Using IN Cell Analyzer 2200" section above.

ROS Measurement Using IN Cell Analyzer 2200 (Caco-2 Cells , HaCaT Cells)

Caco-2 cells were seeded at $1.0 \times 10^2$ cells/mL in a 24-well plate (Corning Incorporated, NY, USA). After 24 hours, ceramides were added to a final concentration of 5 $\mu$M, and culture was performed for 24 hours. HaCaT cells were seeded at $6.0 \times 10^4$ cells/mL in a 96-well plate and incubated for 24 hours, and 50 $\mu$L of the HaCaT cell culture solution was removed. To this culture solution, 50 $\mu$L of the Caco-2 cell culture solution was added. After 48 hours, the ROS in the HaCaT cells was measured in the same manner as in the "ROS Measurement Using IN Cell Analyzer 2200" section above.

#### Effect of Ceramide on Repair of UV Damage in UVB-induced Senescent Cells (Caco-2 Cells → HaCaT Cells)

Caco-2 cells were seeded at $1.0 \times 10^2$ cells/mL in a 24-well plate (Corning Incorporated, NY, USA). After 24 hours, ceramides were added to a final concentration of 5 $\mu$M, and culture was performed for 24 hours.
Subconfluent HaCaT cells were irradiated with UVB at 10 mJ/cm$^2$ and seeded at $6.0 \times 10^4$ cells/mL in a 96-well plate. After 24 hours, 50 $\mu$L of the HaCaT cell culture solution was removed, and 50 $\mu$L of the Caco-2 cell culture solution was added thereto. After 48 hours, the mitochondrial number and area, as well as the mitochondrial membrane potential activity, in the HaCaT cells were measured in the same manner as in the "Effect of Ceramide on Repair of UV Damage in UVB-induced Senescent Cells" section above.

#### Measurement of Transepithelial Electrical Resistance (TER)

A Cell Culture Insert (Falcon) was placed in a 24-well plate. Caco-2 cells were seeded at $2.0 \times 10^5$ cells/well, and 48 hours later, carnosine was added to a concentration of 10 mM. The addition was performed at 3-day intervals, and culture was performed for 2 weeks.
A Millicell ERS-2 (Nihon Millipore K.K., Tokyo) was used to measure the transepithelial electrical resistance. Before the experiment, electrodes were set for use with the Millicell ERS-2 and immersed in a 70% ethanol solution for 10 minutes for sterilization. The electrodes were then dried and immersed in DMEM medium. Next, the electrodes were inserted inside and outside of the Cell Culture Insert, and measurement was performed. The measured values were calculated with reference to the following formula.

$$\text{TER } (\Omega \cdot cm^2) = (\text{measured value} - \text{blank}) \times (\text{insert area})$$

Analysis Results

#### Measurement of Mitochondrial Activity by Using IN Cell Analyzer 2200

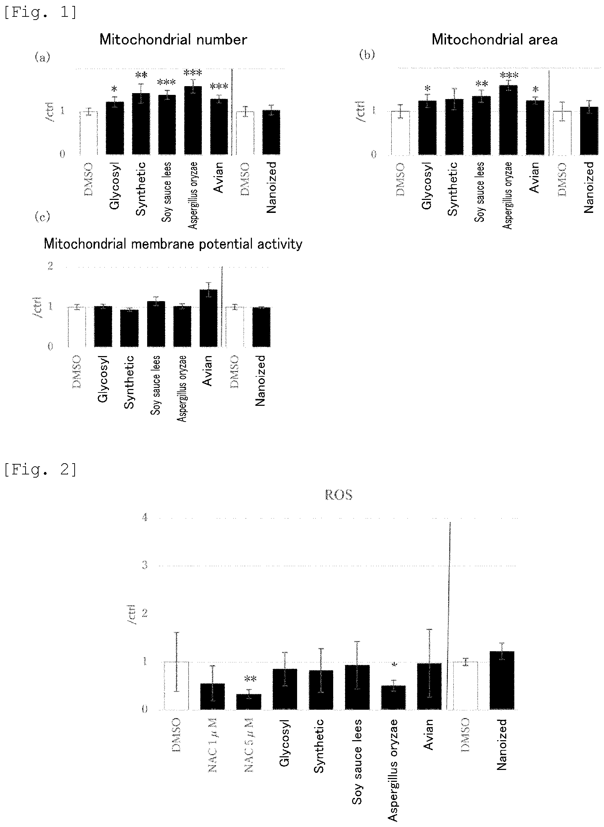

The mitochondrial number and area (MitoTracker Green), as well as the mitochondrial membrane potential activity (MitoTracker Red), per cell were measured using an IN Cell Analyzer 2200 in HaCaT cells treated with ceramides for 48 hours. The glycosylceramide, synthetic ceramide, soy sauce lees ceramide, *Aspergillus oryzae* ceramide, avian ceramide, and non-nanoized ceramide significantly increased the mitochondrial number in HaCaT. The glycosylceramide, soy sauce lees ceramide, *Aspergillus oryzae* ceramide, and avian ceramide significantly increased the mitochondrial area in HaCaT (Fig. 1).

#### ROS Measurement by Using IN Cell Analyzer 2200

After HaCaT cells were irradiated with UVB at 10 mJ/cm$^2$ and then treated with ceramides for 24 hours, the ROS levels were measured using an IN Cell Analyzer 2200. The results indicated that only the *Aspergillus oryzae* ceramide significantly reduced the ROS (Fig. 2).

#### Effect of Restoration of Mitochondrial Activity by Using IN Cell Analyzer 2200

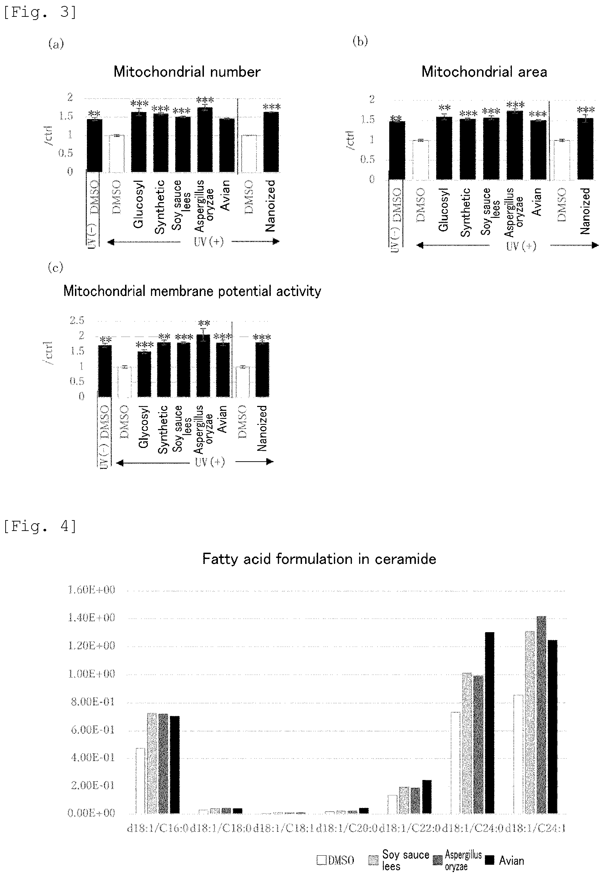

After HaCaT cells were irradiated with UVB at 10 mJ/cm$^2$ and then treated with ceramides for 48 hours, the mitochondrial number and area (MitoTracker Green), as well as the mitochondrial membrane potential activity (MitoTracker Red), per cell were measured using an IN Cell Analyzer 2200 (Fig. 3).

#### Evaluation of Human Ceramide Synthesis of Natural Human Ceramide by LC-MS

Subconfluent HaCaT cells were treated with ceramides for 48 hours, and the fatty acid formulation in the HaCaT cells was measured by LC-MS. The results indicated that HaCaT cells to which the soy sauce lees ceramide, *Aspergillus oryzae* ceramide, or avian ceramide was added showed a significant increase in human ceramides having a long-chain fatty acid containing 24 carbon atoms (Fig. 4).

Measurement of Mitochondrial Activity by Using IN Cell Analyzer 2200 (Caco-2 Cells , HaCaT Cells)

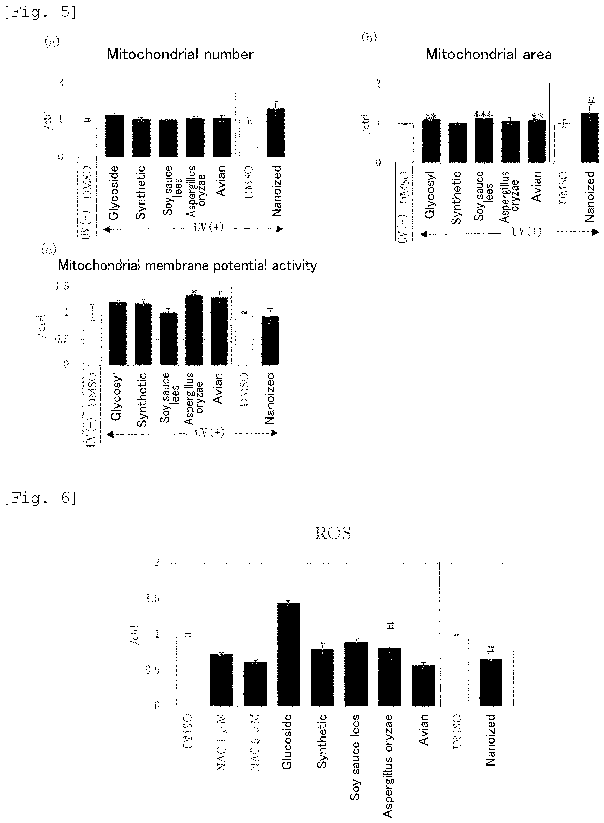

Caco-2 cells were treated with ceramides for 24 hours, and the supernatant thereof was added to HaCaT cells and treated for 48 hours. Then, the mitochondrial number and area (MitoTracker Green), as well as the mitochondrial membrane potential activity (MitoTracker Red), per cell were measured using an IN Cell Analyzer 2200 (Fig. 5).

ROS Measurement by Using IN Cell Analyzer 2200 (Caco-2 Cells→ HaCaT Cells)

Caco-2 cells were treated with ceramides for 24 hours. The supernatant thereof was added to HaCaT cells that had been irradiated with UVB at 10 mJ/cm$^2$, and treated for 24 hours. Then, the ROS levels were measured using an IN Cell Analyzer 2200. The results indicated that the *Aspergillus oryzae* ceramide and nanoized ceramide significantly reduced the ROS (Fig. 6).

Effect of Restoration of Mitochondrial Activity by Using IN Cell Analyzer 2200 (Caco-2 Cells → HaCaT Cells)

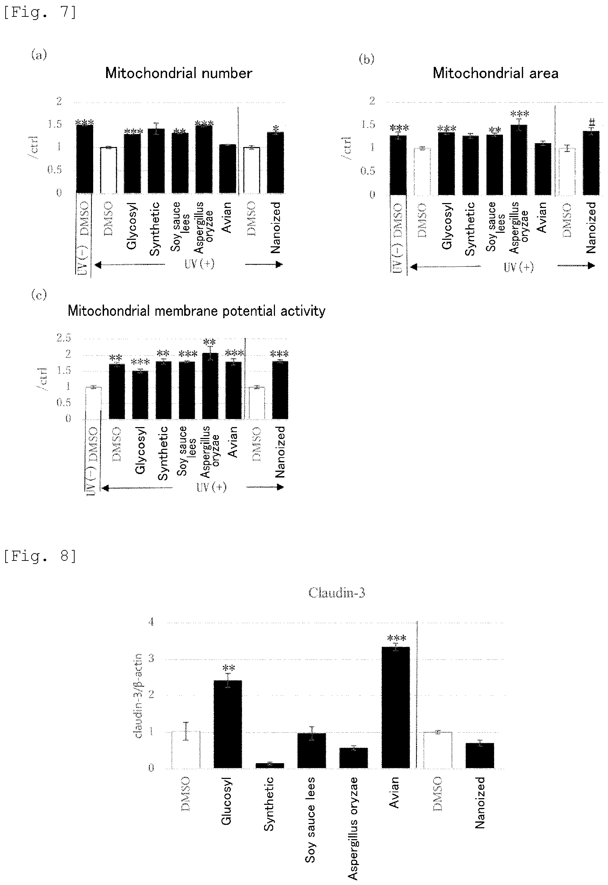

Caco-2 cells were treated with ceramides for 24 hours. The supernatant thereof was added to HaCaT cells that had been irradiated with UVB at 10 mJ/cm$^2$, and treated for 24 hours. Then, the mitochondrial number and area (MitoTracker Green), as well as the mitochondrial membrane potential activity (MitoTracker Red), per cell were measured using an IN Cell Analyzer 2200 (Fig. 7).

Evaluation of Enhancement of Barrier Function of Natural Human Ceramide in CaCo-2 Cells

Measurement of Endogenous Claudin-3 Expression Level (Caco-2 Cells)

Caco-2 cells were treated with ceramides for 24 hours, and the endogenous Claudin-3 expression levels were measured by quantitative RT-PCR. As a result, Claudin-3 was significantly enhanced in the Caco-2 to which the glycosylceramide or avian ceramide was added (Fig. 8).

**Claims**

1. An ethanol-extracted ceramide composition from chicken legs.

2. The composition according to claim 1, comprising ω-O-acyl ceramide.

3. The composition according to claim 2, wherein the ω-O-acyl ceramide present in the composition is of type EOS, and the terminal acyl group is a linoleic acid group.

4. The composition according to claim 2 or 3, wherein 0.1 to 2 mass% of the ceramide present in the composition is w-O-acyl ceramide.

5. The composition according to claim 1 or 2, which is for enhancing an epithelial barrier.

6. The composition according to claim 1 or 2, which is for enhancing a skin barrier or enhancing an intestinal barrier.

7. The composition for enhancing a skin barrier according to claim 6, which is for taking care of skin damage caused by ultraviolet rays.

8. The compositions for enhancing an intestinal barrier according to claim 6, which is for enhancing intestinal epithelial tight junctions.

9. The composition according to claim 1 or 2, wherein 90 mass% or more of the ceramide present in the composition is a free ceramide with a ceramide skeleton that is a combination of a sphingoid base containing 2 hydroxyl groups, 18 carbon atoms, and 1 carbon-carbon double bond; and a fatty acid containing 16 to 34 carbon atoms, 0 or 1 carbon-carbon double bond, and 0 hydroxyl groups.

10. The composition according to claim 1 or 2, wherein 90 mass% or more of the ceramide present in the composition is ceramide NS.

[Fig. 1]

(a) Mitochondrial number

(b) Mitochondrial area

(c) Mitochondrial membrane potential activity

[Fig. 2]

ROS

[Fig. 3]

(a)

**Mitochondrial number**

(b)

**Mitochondrial area**

(c)

**Mitochondrial membrane potential activity**

[Fig. 4]

**Fatty acid formulation in ceramide**

[Fig. 5]

(a) Mitochondrial number

(b) Mitochondrial area

(c) Mitochondrial membrane potential activity

[Fig. 6]

ROS

[Fig. 7]

(a)

Mitochondrial number

(b)

Mitochondrial area

(c)

Mitochondrial membrane potential activity

[Fig. 8]

Claudin-3

[Fig. 9]

## INTERNATIONAL SEARCH REPORT

| | International application No. |
|---|---|
| | **PCT/JP2022/031631** |

### A. CLASSIFICATION OF SUBJECT MATTER

*A61K 31/164*(2006.01)i; *A23L 33/10*(2016.01)i; *A61K 8/68*(2006.01)i; *A61K 9/107*(2006.01)i; *A61K 35/57*(2015.01)i; *A61K 36/06*(2006.01)i; *A61P 1/00*(2006.01)i; *A61P 43/00*(2006.01)i; *A61Q 19/08*(2006.01)i
FI:  A61K31/164; A61K8/68; A61Q19/08; A61P1/00; A61P43/00 105; A61K36/06 A; A61K35/57; A61K9/107; A23L33/10

According to International Patent Classification (IPC) or to both national classification and IPC

### B. FIELDS SEARCHED

Minimum documentation searched (classification system followed by classification symbols)

A61K31/164; A23L33/10; A61K8/68; A61K9/107; A61K35/57; A61K36/06; A61P1/00; A61P43/00; A61Q19/08

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | KR 10-1645917 B1 (YANG, Hyeong-Yeol) 12 August 2016 (2016-08-12) claims, examples | 1-4, 9-10 |
| A | | 5-8 |
| X | NATA et al. Influence of Soy Protein Isolate on Gelatin-based Edible Film Properties. MATEC Web of Conferences. 2018, vol. 156, Article Number 01014, pp. 1-5, <DOI:10.1051/matecconf/201815601014> in particular, abstract, p. 1, left column, lines 1-2, p. 2, left column, [2.2. Preparation of gelatin from chicken leg skin] | 1-4, 9-10 |
| A | | 5-8 |
| A | JP 2020-97539 A (GENUINE R&D CO LTD) 25 June 2020 (2020-06-25) claims, paragraphs [0002], [0059] | 1-10 |
| A | WO 2019/049964 A1 (GENUINE R&D CO LTD) 14 March 2019 (2019-03-14) claims, examples | 1-10 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **20 October 2022** | **08 November 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

INTERNATIONAL SEARCH REPORT
Information on patent family members

International application No.

**PCT/JP2022/031631**

| Patent document cited in search report | | | Publication date (day/month/year) | Patent family member(s) | Publication date (day/month/year) |
|---|---|---|---|---|---|
| KR | 10-1645917 | B1 | 12 August 2016 | (Family: none) | |
| JP | 2020-97539 | A | 25 June 2020 | US 2022/0047491 A1 claims, paragraphs [0002], [0080] EP 3912684 A1 CN 113260422 A KR 10-2021-0104734 A | |
| WO | 2019/049964 | A1 | 14 March 2019 | (Family: none) | |

Form PCT/ISA/210 (patent family annex) (January 2015)

**REFERENCES CITED IN THE DESCRIPTION**

**Patent documents cited in the description**

- JP 2012126910 A

- WO 2019049964 A

**Non-patent literature cited in the description**

- **ROBSON K. J. et al.** *J. lipid Res.,* 1994, vol. 35, 2060

- Dictionary of Pharmaceutical Additives. Yakuji Nippo, Limited, 2016